(19) Europäisches Patentamt
European Patent Office
Office européen des brevets

(11) Veröffentlichungsnummer: **0 098 945**
**B1**

(12) # EUROPÄISCHE PATENTSCHRIFT

(45) Veröffentlichungstag der Patentschrift: **25.09.85**

(51) Int. Cl.[4]: **C 07 C 69/738**

(21) Anmeldenummer: **83105083.6**

(22) Anmeldetag: **21.05.83**

(54) **Verfahren zur Herstellung von enantiomerenreinem Methyljasmonat.**

(30) Priorität: **29.05.82 DE 3220385**

(43) Veröffentlichungstag der Anmeldung: **25.01.84 Patentblatt 84/4**

(45) Bekanntmachung des Hinweises auf die Patenterteilung: **25.09.85 Patentblatt 85/39**

(84) Benannte Vertragsstaaten: **AT BE CH DE FR GB IT LI LU NL SE**

(56) Entgegenhaltungen:
**ANGEWANDTE CHEMIE, Band 92, Br. 12, 1980, Weinheim-Bergstrae G. QUINKERT et al. "Ein photochemischer Weg zu (+-)-Östron" Seiten 1060-1062**
**ANGEWANDTE CHEMIE, Band 92, Nr. 12, 1980 Weinheim-Bergstrae G. QUINKERT et al. "Asymmetrische Totalsynthese von (+-)-Östron" Seiten 1062-1063**
**Chemical Abstracts Band 95, Nr. 13, 1981 Columbus, Ohio, USA Seite 642, Spalte 2, Abstract Nr. 114895n**

(73) Patentinhaber: **HOECHST AKTIENGESELLSCHAFT, Postfach 80 03 20, D-6230 Frankfurt am Main 80 (DE)**

(72) Erfinder: **Quinkert, Gerhard, Prof. Dr., Schauinsland 32, D-6246 Glashütten/Taunus (DE)**
Erfinder: **Dürner, Gerd, Dr., Zeisselstrasse 20, D-6000 Frankfurt am Main (DE)**
Erfinder: **Adam, Friedhelm, Dr., Scheffelstrasse 24, D-6000 Frankfurt am Main (DE)**

**EP 0 098 945 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist ein Verfahren, das durch eine asymmetrische Synthese erstmalig die Herstellung von enantiomerenreinem Methyljasmonat erlaubt.

Aus Anwandte Chemie *92*, 1060-1062/1063 [1980] ist es bekannt, durch Umsetzung von Malonsäureestern mit mindestens einem Chiralitätszentrum im Alkoholrest an 1,4-Dibrom-2-buten Cyclopropanderivate mit induzierter Asymmetrie herzustellen, die nach diesen beiden Literaturstellen über mehrere weitere Stufen zu Östronderivaten umgesetzt werden. Hinweis oder Anregungen, dass enantiomerenreines Methyljasmonat hergestellt werden könnte, finden sich darin jedoch nicht.

Methyljasmonat ist eine in den Riechstoffindustrie begehrte Verbindung. Auf die vielen hierfür bereits entwickelten Herstellungsverfahren weisen R.V. Stevens und N. Hrib in Terahedron Letters, 1981, 4791 hin. Diese bekannten Synthesen von Methyljasmonat haben jedoch stets zu einem Gemisch zweier Razemate geführt. Um die mühsame Auftrennung dieses Gemisches zu umgehen, wurde deshalb nach einem Verfahren gesucht, das die Herstellung von enantiomerenreinem Methyljasmonat der Formel I:

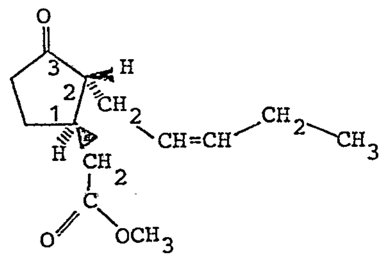

(I)

erlaubt. Diese Aufgabe wurde durch ein mehrstufiges Verfahren gelöst, bei dem man:

a) einen Malonester, dessen Alkoholrest X mindestens ein Chiralitätszentrum aufweist, mit 1,4-Dihalogen-2-buten unter Phasentransferbedingungen zur Verbindung der Formel II:

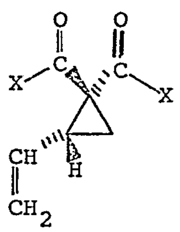

(II)

umsetzt,

b) hieraus die beim Erhitzen in einem Lösungsmittel in einer Gleichgewichtsreaktion entstehende diastereomeren Verbindung der Formel III (a) abtrennt und in den entsprechenden enantiomeren Dimethylester der Formel IIIb:

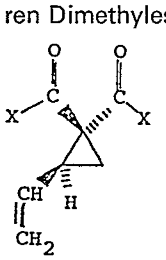

(IIIa)

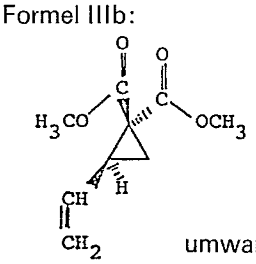

(IIIb)

umwandelt,

c) den Dimethylester IIIb mit 2-Pentinylmalonsäuredimethylester unter alkalischen Bedingungen zu einem Cyclopentanonderivat umsetzt, aus dem nach Hydrolyse und Decarboxylierung die Verbindung der Formel IV:

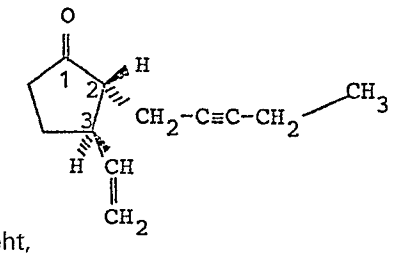

(IV)

entsteht,

d) deren Vinylgruppe man durch Wasseranlagerung, Oxidation zur endständigen Carboxylgruppe und Veresterung zur Verbindung der Formel:

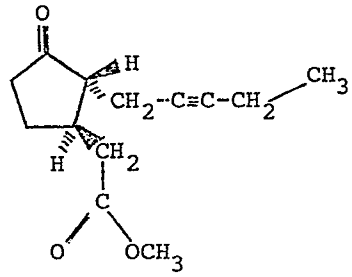

(V)

umsetzt, aus der dann durch Wasserstoffanlagerung an die Butinylgruppe das Methyljasmonat gebildet wird.

Ausgangsverbindung für die Synthese ist ein Malonsäureester, dessen Alkoholrest mindestens ein Chiralitätszentrum aufweist. Bevorzugt werden jedoch Alkohole, die 2 oder sogar 3 Chiralitätszentren haben. Ganz besonders hat sich der Alkoholrest gemäss Formel VI:

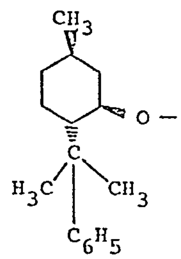

(VI)

bewährt. Ein Malonester, der einen derartigen Alkoholrest enthält, wird dann in einer die Asymmetrie induzierenden Reaktion mit 1,4-Dihalogen-2-buten zur Verbindung II umgesetzt, in der X z. B. für einen Substituenten der Formel VI steht. Erhitzt man die Verbindung II in einem Lösungsmittel wie p-Cymol unter Rückfluss, dann stellt sich ein Gleichgewicht zwischen Verbindung II und der diastereomeren Verbindung IIIa im Verhältnis 2,5:1 ein. Die Verbindung IIIa lässt sich durch präparative Hochdruckflüssigkeitschromatographie (= HPLC) abtrennen. Durch mehrfache Wiederholung der Reaktion kann die Ausbeute an Verbindung IIIa erheblich gesteigert werden.

Die Verbindung IIIa wird dann verseift, in IIIb umgegesetzt und durch Umsetzung mit 2-Pentinylmalonsäuredimethylester unter alkalischen Bedingungen in ein Cyclopentanonderivat umgewandelt, das nach Hydrolyse und Decarboxylierung die Verbindung IV ergibt, die durch Destilla-

tion und Säulenchromatographie rein hergestellt wird. Geringe Mengen der hierbei entstehenden, am Kohlenstoffatom-2 epimeren Verbindung stören die folgenden Verfahrensschritte nicht.

Die Verbindung IV wird anschliessend zum Beispiel mit dem 9-Borabicyclo-[3.3.1]-nonan reduziert und dabei gleichzeitig an die Vinylseitengruppe Wasser angelagert. Der entstandenen Alkohol wird dann mit Oxidationsmitteln in die Carboxylgruppe übergeführt und hieraus zum Beispiel mit Diazomethan der Methylester hergestellt. Durch diese Reaktionsschritte lässt sich die Verbindung V herstellen, die dann vorzugsweise mit Lindlar-Katalysator bei Raumtemperatur katalytisch hydriert wird. Das entstehende Produkt enthält die Verbindung der Formel I zu 95%, daneben aber auch die am Kohlenstoffatom-2 epimere Verbindung in einer Menge von 5%. Durch HPLC wird die Verbindung I in reiner Form gewonnen.

Das beschriebene Verfahren hat ausserdem den Vorzug, mit der Verbindung II in quantitativer optischer Ausbeute ein Zwischenprodukt zu liefern, aus dem andere chirale 2-Vinyl-cyclopropan-1,1-dicarbonsäurediester in extremer Reinheit hergestellt werden können, die völlig frei von nachweisbarem Razemat sind. Ein auf diesem Weg gewonnener Ester der Formel VII:

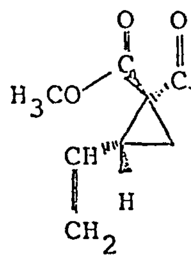

(VII)

eröffnet den Weg zu einer stereospezifischen, asymmetrischen Totalsynthese verschiedener chiraler Produkte, z. B. auch des östrons, nach dem in der DE-OS Nr. 3014120 beschriebenen Verfahren. Während mit den bekannten Verfahren die Verbindung VII nur mit $[\alpha]_D^{20}$ – + 43,9 in $CCl_4$, d. h. in einer optischen Ausbeute von etwa 80% herstellbar ist (Angew. Chem. 92 (1980), 1063), konnte jetzt diese Verbindung VII mit $[\alpha]_D^{20}$ – + 55,2 in $CCl_4$ gewonnen und damit der Wert der Referenzsubstanz erreicht werden.

Die Tatsache, dass es erstmals gelungen ist, den chiralen Baustein der Formel II und damit auch die Verbindung der Formel VII in einer asymmetrischen Synthese in 100%ig. optische Ausbeute zu erhalten, muss als entscheidender Fortschritt gegenüber dem Stand der Technik angesehen werden.

Die Erfindung wird durch die nachfolgenden Beispiele erläutert, wobei anstelle von $[\alpha]_D^{20}$ die Bezeichnung $\alpha(\lambda)$ = 589 nm verwendet wird.

*Herstellung der Verbindung II (mit X = Formel VI)*

67,5 g des Malonsäurediesters, der den Alkoholrest der Formel VI enthält [$\alpha(\lambda)$: 7,7 (589), 69.7 (365 nm) bei 20°C in n-Hexan], 27,15 g 1,4-Dibrom-2-buten und 2 g Tricaprylmethylammoniumchlorid werden in 200 ml n-Hexan und 100 ml einer 50%ig. NaOH-Lösung 14 Stunden lang bei Raumtemperatur gerührt. Durch präparative HPLC wird die entstandene Verbindung II gereinigt und in einer Ausbeute von 66,5% der Theorie gewonnen. [$\alpha(\lambda)$: 10,0 (589), 55,6° (365 nm) bei 20°C in n-Hexan].

*Verbindung III(a) aus Verbindung II*

10 g der Verbindung II werden in 110 ml p-Cymol 13 Stunden unter Rückfluss erhitzt. Die dabei entstandene Verbindung IIIa wird durch präparative HPLC abgetrennt, der Rückstand erneut in p-Cymol erhitzt. Nach fünfmaliger Wiederholung dieser Prozedur war die Verbindung IIIa in einer Ausbeute von 57% der Theorie erhalten.

[$\alpha(\lambda)$: −3,0 (589), −3,0 (578), −2,6 (546), 3,4 (436), 23,0° (365 nm) bei 20°C in n-Hexan].

*Verbindung IIIb aus Verbindung IIIa*

15,5 g der Verbindung IIIa werden in 150 ml Ethanol gelöst und dazu 30 g KOH und 40 ml Wasser, dem eine Spatelspitze Hydrochinon zugegeben wurde, vermischt und 16 Stunden unter Rückfluss gekocht. Das erhaltene Produkt wurde in Ether aufgenommen und mit einem Überschuss von Diazomethan versetzt. Anschliessend wurde durch Kugelrohrdestillation und Säulenchromatographie (Kieselgel) gereinigt und die Verbindung IIIb in 72%ig. Ausbeute enantiomerenrein gewonnen [$\alpha(\lambda)$: −54.9° (589 nm) bei 25°C in $CCl_4$].

*Verbindung IV aus Verbindung IIIb*

3,2 g 2-Pentinylmalonsäure-dimethylester wurden in 5 ml Methanol gelöst und mit 380 mg Natrium in 25 ml Methanol 10 min lang bei 60°C zusammen gegeben. Dann wurden 3 g der Verbindung IIIb in 5 ml Methanol zugefügt und 10 min bei 60°C unter Rühren stehengelassen. Dann wurde das Lösungsmittel entfernt und die Mischung eine Stunde auf 130°C erwärmt.

Nach einer Grobreinigung durch Kugelrohrdestillation wurden 3 g des grob gereinigten Rohproduktes in 10 ml Methanol aufgenommen und zu einer Lösung von 3,5 g NaOH in 75 ml $H_2O$ gegeben und 20 Stunden bei Raumtemperatur sowie anschliesend 16 Stunden unter Rückfluss umgesetzt. Danach wurde mit konzentrierter HCL gegen Bromthymolblau neutralisiert. Anschliessend wurde im Verlauf einer Stunde 18 g $NaH_2PO_4 \cdot 2H_2O$ zugegeben und unter Eiskühlung 4 Stunden gerührt. Die Verbindung der Formel IV wurde durch Säulenchromatographie (Kieselgel) und Kugelrohrdestillation gereinigt. Die Ausbeute betrug 24% der Theorie. Das Verhältnis der Verbindung IV zu der am Kohlenstoffatom-2 epimeren Verbindung betrug 95:5.

[$\alpha(\lambda)$: −70,1 (589 nm), −75,2 (578), −89,5 (546), −213,7 (436), −578,4° (365 nm) bei 20°C in Methanol.

$\Theta(\lambda)$: −8575 (296), −8535 (306), −4590 (318 nm; Schulter) bei 20°C in Dioxan].

*Verbindung V aus Verbindung IV*

525 mg der Verbindung IV wurden in 15 ml Tetrahydrofuran gelöst dazu 6,5 M 9-Borabicyclo-[3.3.1]-nonan in 13 ml Tetrahydrofuran getropft und dann 2 Stunden bei Raumtemperatur gerührt. Dann wurden 4 ml einer 3-n-NaOH zugegeben und unter Eiskühlung 4 ml einer 30%ig. $H_2O_2$-Lösung zugetropft und nochmal 2 Stunden bei Raumtemperatur gerührt. Danach wurde das Produkt durch Kugelrohrdestillation gereinigt, in 50 ml Ether gelöst und 10 ml des Jones-Reagenzes unter Eiskühlung zugegeben und danach 14 Stunden bei Raumtemperatur gerührt. Nach Entfernung des überschüssigen Oxidationsmittels und Neutalisation wurde ein Überschuss an Diazomethan zugegeben und nach Kugelrohrdestillation, gefolgt von einer Säulenchromatographie (Kieselgel) und erneuter Kugelrohrdestillation die Verbindung der Formel V in einer Ausbeute von 65% der Theorie isoliert.

[α(λ): 84,7 (589 nm), −589,7° (365 nm) bei 20°C in Methanol.

Θ(|): −9490 (297), −8860 (302, Minimum), −9250 (306), −5030 (317 nm; Schulter) bei 20°C in Dioxan].

*Verbindung I aus Verbindung V*

200 mg der Verbindung V wurden in 25 ml Petrolether über 50 mg Lindlar-Katalysator bei Raumtemperatur katalytisch hydriert. Das erhaltene Produkt wurde nach 30 min durch Säulenchromatographie (Kieselgel) und Kugelrohrdestillation isoliert. Es wurde ein Gemisch erhalten, das zu 95% aus der Verbindung I und zu 5% aus der am Kohlenstoffatom-2 epimeren Verbindung bestand. Nach Abtrennung des cis-Isomeren durch HPLC wurde die Verbindung I enantiomerenrein gewonnen. Die genaue chemische Bezeichnung von Verbindung I ist (Z, 1R, 2R)-3-oxo-(2-pentenyl-cyclopentyl)-essigsäuremethylester. Sie ist durch die folgenden optischen Daten gekennzeichnet:

α(λ): −90,2 (589 nm), −95,8 (578), −113,4 (546), −252,3 (436), −623,4° (365 nm) bei 20°C in Methanol.

Θ(λ): −9650 (297), −9050 (302; Minimum), −9475 (306), −5555 (315 nm; Schulter) bei 20°C in Dioxan.

*Verbindung VII aus Verbindung II*

15,5 g der Verbindung II werden in 150 ml Ethanol gelöst und dazu 30 g KOH und 40 ml Wasser, dem eine Spatelspitze Hydrochinon zugegeben wurde, vermischt und 16 Stunden unter Rückflussgekocht. Das erhaltene Produkt wurde in Ether aufgenommen und mit einem Überschuss von Diazomethan versetzt. Anschliessend wurde durch Kugelrohrdestillation und Säulenchromatographie (Kieselgel) gereinigt und die Verbindung VII in 65%ig. Ausbeute enantiomerenrein gewonnen.

[α(λ): +55,2° (589 nm) bei 25°C in $CCl_4$].

**Patentanspruch**

Verfahren zur Herstellung von enantiomerenreinem Methyljasmonat der Formel I:

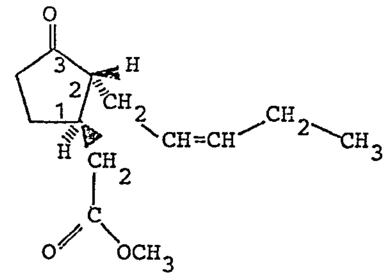

(I)

dadurch gekennzeichnet, dass man

a) einen Malonester, dessen Alkoholrest X mindestens ein Chiralitätszentrum aufweist, mit 1,4-Dihalogen-2-buten unter Phasentransferbedingungen zur Verbindung der Formel II:

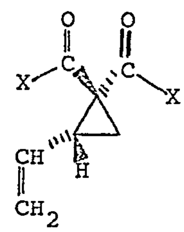

(II)

umsetzt,

b) hieraus die beim Erhitzen in einem Lösungsmittel in einer Gleichgewichtsreaktion entstehende diastereomere Verbindung der Formel IIIa abtrennt und in den entsprechenden enantiomeren Dimethylester der Formel IIIb:

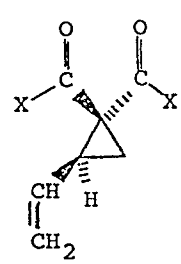

(IIIa)

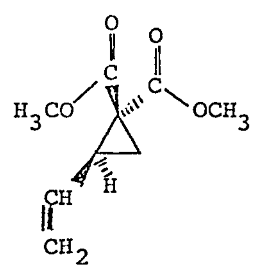

(IIIb)

umwandelt,

c) den Dimethylester IIIb mit 2-Pentinylmalonsäuredimethylester unter alkalischen Bedingungen zu einem Cyclopentanonderivat umsetzt, aus dem nach Hydrolyse und Decarboxylierung die Verbindung der Formel IV:

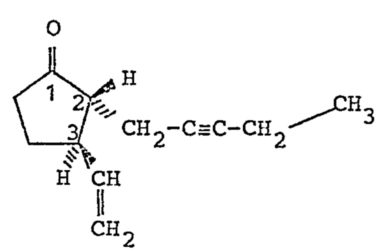

(IV)

entsteht,

d) deren Vinylgruppe man durch Wasseranlagerung, Oxidation zur endständigen Carboxylgruppe und Veresterung zur Verbindung der Formel V:

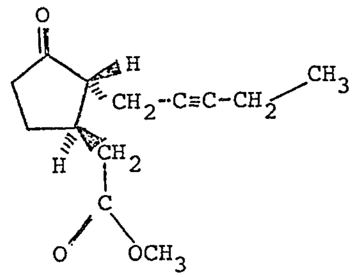

(V)

umsetzt, aus der dann durch Wasserstoffanlagerung an die Butinylgruppe das Methyljasmonat gebildet wird.

## Revendication

Procédé de préparation du jasmonate de méthyle sous forme d'énantiomère pur de formule I:

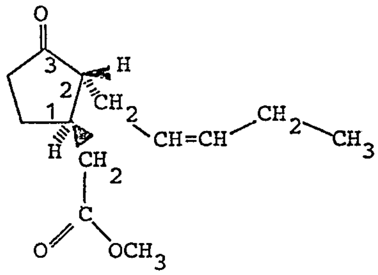

(I)

caractérisé par le fait que l'on:

a) fait réagir un ester malonique dont le résidu alcool X présente au moins un centre de chiralité avec du 1,4-dihalogéno-2-butène dans des conditions de transfert de phases, pour aboutir au composé de formule II:

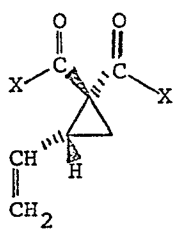

(II)

b) sépare de celui-ci un composé diastéréomère de formule IIIa qui se forme par chauffage dans un solvant dans une réaction d'équilibre et que l'on transforme en ester de diméthyle énantiomère correspondant de formule IIIb:

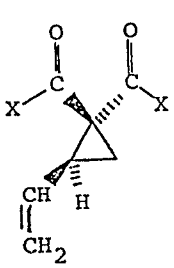

(IIIa)

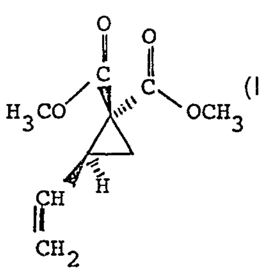

(IIIb)

c) fait réagir l'ester de diméthyle IIIb avec du 2-pentynylmalonate de diméthyle dans des conditions alcalines pour arriver à un dérivé cyclopentanone à partir duquel se forme, après hydrolyse et décarboxylation, le composé de formule IV:

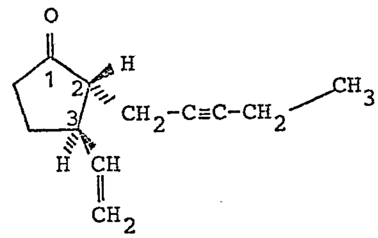

(IV)

d) dont on transforme le groupe vinyle par hydratation, oxydation, en groupe carboxy et on le transforme par estérification en composé de formule V:

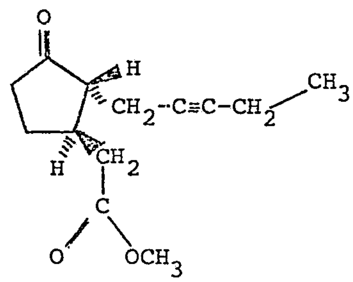

(V)

à partir duquel le jasmonate de méthyle est formé par addition d'hydrogène sur le groupe butynyle.

## Claim

A process for the preparation of enantiomerically pure methyl jasmonate of the formula I:

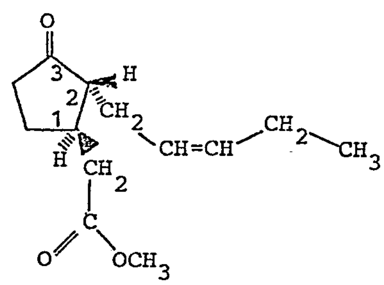

(I)

which comprises:

(*a*) reacting a malonic ester, the alcohol moiety X of which has at least one center of chirality, with 1,4-dihalogeno-2-butene under phase-transfer conditions to give a compound of the formula II:

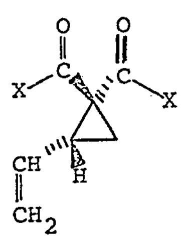

(II)

(*b*) separating out of this the diastereomeric compound of the formula IIIa, formed in an equilibrium reaction by heating in a solvent, and converting it into the corresponding enantiomeric dimethyl ester of the formula IIIb:

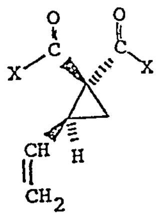 (IIIa) 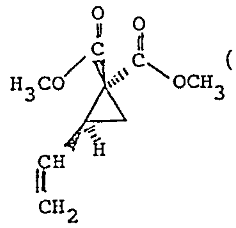 (IIIb)

(*c*) reacting the dimethyl ester IIIb with dimethyl 2-pentynylmalonate under alkaline conditions to give a cyclopentanone derivative from which, after hydrolysis and decarboxylation, the compound of the formula IV is produced:

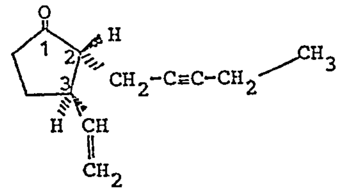 (IV)

(*d*) reacting its vinyl group by addition of water, oxidation to a terminal carboxyl group and esterification to give the compound of the formula V:

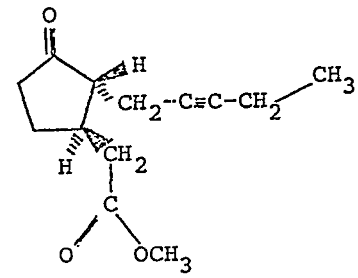 (V)

from which methyl jasmonate is formed by addition of hydrogen to butynyl group.